# EUROPEAN PATENT APPLICATION

(11) **EP 4 574 187 A1**
(43) Date of publication of application: **25.06.2025**
(21) Application number: 23218496.0
(22) Date of filing: 20.12.2023
(51) Int. Cl.: A61M 5/168

(54) **SYSTEM, PUMP, LINE AND METHOD FOR MONITORING PRESSURE IN A LINE OF A MEDICAL FLUID DELIVERY SYSTEM**

(71) Applicant: Fresenius Kabi Deutschland GmbH, 61352 Bad Homburg (DE)
(72) Inventor: Baumgart, Steffen, 36088 Hünfeld (DE); Pütter, Harry, 36364 Bad Salzschlirf (DE)
(74) Representative: Fresenius Kabi Deutschland GmbH

(57) **Abstract**

A system (1) for monitoring pressure in a line of a medical fluid delivery system is provided. The system (1) comprises a flow-through element (11) to be brought into fluid communication with a line of a medical fluid delivery system, wherein the volume of the flow-through element (11) is fixed, a probe (12) provided inside the flow-through element (11), wherein the probe volume (V) is responsive to the pressure (P) in the flow-through element (11), and an ultrasonic measurement unit (13) for detecting a current probe volume (V) and/or detecting a change in probe volume (V). A line of a medical fluid delivery system, a pump for use with a line of a medical fluid delivery system and a method for monitoring pressure in a line of a medical fluid delivery system are likewise provided.

## Description

The invention relates to a system for monitoring pressure in a line of a medical fluid delivery system, in particular an intravenous line, a pump for use with a line of medical fluid delivery system, as well as a line of a medical fluid delivery system and a method for monitoring pressure in a line of a medical fluid delivery system, in particular in an intravenous line.

Medical fluid delivery systems deliver medical fluids such as medication, anaesthetics and/or nutrition to a body of a patient. Such systems conventionally comprise a pump which administers the respective medical fluid to the patient and a line which connects the pump and an entry point through which the medical fluid is administered into the patient body.

The medical fluid delivery system is, for example, an intravenous (IV) or intra-arterial administration system for administering medical fluids directly into a vein or an artery of a patient. The entry point is hence in the vein or artery of a patient, which is accessed e.g. by piercing the vein or artery with a needle or a cannula and connecting a syringe or tube connected to the needle or the cannula directly to the intravenous line, or IV line for short, or intra-arterial line. Alternatively, the medical fluid delivery system is an enteral feeding system for administering medical fluids directly into the gastrointestinal tract of a patient. The entry point depends on whether oral, gastric or rectal enteral feeding is provided. In the case of gastric feeding, for example, the entry point is the stomach and the enteral feeding line connects an enteral feeding catheter placed in the stomach with an enteral feeding pump.

Medical fluid delivery systems with corresponding lines have in common, that a pressure in the line of the delivery systems needs to be monitored. e.g. to detect clogging of the line or leaks in the line as well as other irregularities such air trapped in the line. To this end, a system for pressure monitoring needs to be provided.

Medical fluid pumps, in particular IV pumps, conventionally comprise a tube section along which a medical fluid is transported by successive pinching of said tube section in order to provide the pumping effect. Some pumps comprise exchangeable cassettes which contain the tube section used for providing the pumping effect. In order to enable pressure monitoring, current medical fluid pumps, in particular IV pumps, comprise pressure sensors arranged on pump tubing, in particular on the tube section providing the pumping effect. If the pump is a pump for use with a cassette, pressure sensors are conventionally placed on the tube section providing the pumping effect inside the cassette. Alternatively or additionally, pressure sensors may be provided in the line of the medical fluid delivery system. These pressure sensors are typically placed such that they are in direct contact with medical fluids inside the line or with flexible parts of the line. As a consequence, they may burst if pressure rises. Moreover, as these sensors are generally fixed to the line, they are being disposed whenever the line is being disposed, making the line very expensive and increasing waste.

The pressure measured inside the line depends on the point of measurement. A patient-near pressure measurement yields a different pressure value than a pressure measurement within the delivery system's pump. It is therefore desirable to provide a pressure-monitoring system which allows a flexible measurement along the line.

In view of all these disadvantages of the prior art, it is an object of the instant invention to provide a system and a corresponding method for monitoring pressure in a line of a medical fluid delivery system, in particular in an IV line, which is cost-effective, sustainable, safe and allows for flexible pressure measurements along a line, in particular an IV line.

This object is achieved by means of a system for monitoring pressure in a line of a medical fluid delivery system, in particular in an IV line, comprising the features of claim 1. The system comprises a flow-through element to be brought into fluid communication with a line of a medical fluid delivery system or a flow-through element being an integral part of a line of a medical fluid delivery system, in particular with an IV line, wherein the volume of the flow-through element is fixed. The flow-through element is configured such that a medical fluid can pass through the flow-through element. The flow-through element is, for example, a tube or a pipe or tube or pipe segment to be brought into fluid communication with a line or a tube or a pipe or a tube or pipe segment being part of a line of a medical fluid delivery system. If the flow-through element is an integral part of a line of a medical fluid delivery system, the flow-through element is the part of the line in which the probe is arranged. The system further comprises a probe provided inside the flow-through element, in particular inside the tube or pipe or tube or pipe segment. The probe volume is responsive to the pressure in the flow-through element. That means, the volume of the probe changes whenever the pressure in the flow-through element changes, depending on the sensitivity of the probe. Further, the system comprises an ultrasonic measurement unit for detecting a current probe volume. Alternatively or additionally, the ultrasonic measurement unit is configured to detect a change in the probe volume. Hence, volume changes and/or absolute volumes can be used to determine pressure changes and/or absolute pressures. As the probe volume is indicative of the pressure inside the flow-through element, a pressure inside a line of a medical fluid delivery system, in particular in an IV line, can be monitored when the flow-through element is brought into fluid contact with the line or is an integral part of the line, in particular with the IV line.

If the flow-through element is a flow-through element to be brought into fluid communication with a line, the system, in particular its flow-through element, can be placed in fluid communication with a line, in particular an IV line, at different positions of the line. Hence, the measurement position can be changed and chosen according to requirements and needs. Also, multiple systems can be placed along the line. Moreover, bringing the flow-through element in fluid communication with a line, in particular an IV line, is reversible. Hence, the system can be detached from a line, in particular an IV line, and therefore be reused, saving costs and waste.

The system is able to withstand internal pressures of a medical fluid delivery system, increasing patient safety.

According to one aspect, at least a part of the probe volume is bounded by an elastic membrane. The elastic membrane changes its surface when the pressure in the flow-through element changes. As a consequence, the probe volume changes when the pressure in the flow-through element changes, making the probe volume responsive to pressure changes in the flow-through element.

According to one aspect, the entire probe volume is bounded by an elastic membrane forming a balloon. At a given initial pressure inside the flow-through element, the volume of the probe forming a balloon is an initial probe volume. Upon a pressure increase, the probe volume decreases as the pressure acts on the elastic membrane of the balloon. Upon a pressure decrease inside the flow-through element, the elastic membrane extends, increasing the probe volume. Hence, a probe is provided with a volume responsive to the pressure inside the flow-through element.

In one example, the probe comprises fixation elements for fixing the probe to the flow-through element. In this way, a floating of the probe is prevented. In particular, a floating from the probe out of the flow-through element into a connected line or into the line which it is an integral part of, in particular IV line, is prevented. Moreover, the probe is hence kept in a fixed position relative to the ultrasonic measurement unit. Fixation elements are, for example, straps connecting the probe to the walls of the flow-through element. The fixation elements are, in one example, elastic. This gives the probe some freedom to respond to fluid flow in the flow-through element when in fluid communication with a line, in particular an IV line.

According to another aspect at least part of the probe volume is bounded by an inner surface of the flow-through element. Hence, part of the probe volume is surrounded by an elastic membrane and part of the probe volume is bounded by the comparatively inelastic inner surface of the flow-through element. In this way, only part of the probe can respond to pressure changes through deformation. This allows to limit the region in which the deformation occurs.

According to one aspect, the probe volume is filled with a compressible medium, in particular a gas, e.g. air. At a defined pressure in the flow-through element, the compressible medium occupies a defined volume. Upon pressure increases, the compressible medium is compressed, reducing the probe volume. On the other hand, if the pressure decreases, the compressible medium extends, increasing the probe volume. In this way, a probe response to the pressure in the flow-through element is provided. The compressibility of the medium is chosen, for example, depending on the pressure range to be expected in a line, in particular an IV line. The sensitivity of the probe to pressure changes in the flow-through elements depends in one example on the compressibility of the medium.

According to one aspect, the flow-through element is a tube or a tube section. The tube section is, in one example, part of a line of a medical fluid delivery system. In this case, the flow-through element is an integral part of the line. For example, the tube has a circular cross section of a D-shaped cross section. This makes connecting the flow-through element to a line, in particular an IV line, particularly simple. In one example, the diameter of the tube with circular cross section is given by the diameter of a line of medical fluid delivery system, in particular the diameter of an IV line. For example, the inside diameter is between 1mm and 8mm, preferably 2 and 5mm, in one example 3mm. The outer diameter is, for example, between 2mm and 10mm, preferably 3mm and 6mm, in one example 4,1mm.

According to one aspect, the ultrasonic measurement unit comprises an ultrasonic source and an ultrasonic sensor. The ultrasonic source is configured to emit ultrasonic waves. The ultrasonic sensor is configured to detect ultrasonic waves.

In one example, the ultrasonic measurement unit is configured to measure the travel time of ultrasonic pulses emitted by the ultrasonic source towards the flow-through element and detected by the ultrasonic sensor. Hence, the ultrasonic measurement unit is configured to determine the travel time of ultrasound pulses emitted by the ultrasound source, reflected by the probe and detected by the ultrasound sensor. In particular, the ultrasonic measurement unit is configured to determine a difference in travel times through a change in probe volume, in particular time delays caused by a change in probe volume. In this way, the ultrasonic measurement unit is e.g. configured to detect changes in probe volume. In one example, the ultrasonic measurement unit has information on a reference volume and a reference travel time and is, hence, configured to determine a probe volume based on travel-time or time-delay measurements. Alternatively or additionally, the ultrasonic measurement unit is configured to measure the amplitude of ultrasonic waves emitted by the ultrasonic source towards the flow-through element and detected by the ultrasonic sensor. As the square of the amplitude yields the intensity, the ultrasonic measurement unit is configured to determine the intensity detected at the ultrasonic sensor of ultrasound waves emitted by the ultrasonic source towards the probe. Absorption and reflection of ultrasound waves by the probe influence the detected amplitude, hence intensity. Therefore, the ultrasonic measurement unit is configured to e.g. detect a change in probe volume through the detection of changes in amplitude and/or intensity. In one example, the ultrasonic measurement unit has information on a reference volume and a reference intensity or reference amplitude and is, hence, configured to determine the probe volume based on amplitude and/or intensity of ultrasound waves emitted by the ultrasound source towards the probe and detected by the ultrasound sensor.

According to one aspect, the ultrasonic measurement unit is part of a pump for use with a line of a medical fluid delivery system. For example, the ultrasonic measurement unit is arranged in the pump to be contacted by a line of a medical fluid delivery system when such a line is connected to the pump. In particular, the ultrasonic measurement unit is arranged in the pump such that it is placed on an exterior of a line, in particular a flow-through element being an integral part of a line, when the line is connected to the pump. In particular, the ultrasonic measurement unit is a reusable part of the pump, whereas the flow-through element with the probe forms part of the disposable line.

According to another aspect, the ultrasonic measurement unit is arranged on an exterior of the flow-through element, in particular a flow-through element to be brought into fluid communication with a line of a medical fluid delivery system. Hence, the ultrasonic measurement unit is not in contact with a fluid inside the flow-through element when the flow-through element is in fluid communication with a line, in particular an IV line, of a medical fluid delivery system. For example, the ultrasonic measurement unit and the flow-through element form a single component to be connected with a line of a medical fluid delivery system. In particular, the flow-through element with probe and ultrasonic measurement unit is a disposable, i.e. discarded after use.

In one example, the ultrasonic source and the ultrasonic sensor are arranged on an outer surface of the flow-through element such that ultrasonic waves emitted by the ultrasonic source and reflected by the probe are detected by the ultrasonic sensor. Alternatively, the ultrasonic source and the ultrasonic sensor are placed in a pump for use with a medical fluid delivery system such that the ultrasonic source and the ultrasonic sensor are arranged on an outer surface of the flow-through element being an integral part of a line, when the line is connected to the pump or used with the pump, such that ultrasonic waves, in particular ultrasonic pulses, emitted by the ultrasonic source and reflected by the probe are detected by the ultrasonic sensor. In both cases, the ultrasonic source and the ultrasonic sensor are placed relative to each other and relative to the probe such that the ultrasonic sensor detects ultrasonic waves reflected from the probe. In case that the ultrasonic measurement unit is part of a pump, this applies, if a line of which a flow-through element with the probe is an integral part is connected to the pump or used with the pump. For example, the ultrasonic measurement unit is configured to carry out a run-time or travel-time measurement. In particular, the ultrasonic measurement unit detects changes in travel times of ultrasonic-wave pulses emitted by the ultrasonic source and reflected off the probe and detected by the ultrasonic sensor. The ultrasonic source and sensor are placed relative to the probe such that an increase of the probe volume causes a reduction in travel time, whereas a decrease in probe volume results in an increase of travel time. In this way, the travel time changes are used to infer probe volume changes which are indicative of changes of the pressure in a line in fluid communication with the flow-through element.

Alternatively or additionally, ultrasonic source and sensor are placed in a fluid-flow direction along the flow-through element, such that ultrasound-wave pulses emitted by the ultrasound sensor travel in the direction or opposite the direction of a fluid flowing through the flow-through element when in fluid communication with a line, in particular an IV line, or when being an integral part of a line. In this example, changes in fluid velocity also influence the travel times of ultrasound pulses detected by the ultrasound sensor. Hence, changes in travel times cannot be exclusively assigned to probe volume changes. In an alternative example, ultrasonic source and sensor are placed perpendicular to a fluid-flow direction along the flow-through element, such that ultrasound-wave pulses emitted by the ultrasound sensor travel in a plane perpendicular to the direction of a fluid flowing through the flow-through element when in fluid connection with a line. In this example, the fluid velocity does not influence the travel times of ultrasound pulses detected by the ultrasound sensor.

In an alternative example, the ultrasonic source and the ultrasonic sensor are arranged on an outer surface of the flow-through element such that ultrasonic waves emitted by the ultrasonic source and transmitted through the flow-through element are detected by the ultrasonic sensor. Thus, the ultrasonic source and senor are placed relative to each other and relative to the probe such that ultrasonic waves emitted by the source at least partially transmit the flow-through element and reach the sensor. The remaining part is absorbed or reflected by the probe. The extent of absorption and reflection of the emitted ultrasonic waves changes with the probe volume. Hence, the ultrasonic measurement unit infers the probe volume from a measurement of ultrasonic waves transmitted through the flow-through element. In particular, the ultrasonic measurement unit is configured to measure the changes in intensity of ultrasonic waves detected by the ultrasonic sensor. The intensity is determined by the amplitude of the detected ultrasonic waves. If the probe volume increases, the intensity detected by the sensor decreases and vice versa. The reflection and absorption properties of a probe filled with a compressible medium depend on the choice of compressible medium, in particular its density and hence, its refractive index.

In one example, ultrasonic source and sensor are placed in a fluid-flow direction along the flow-through element, such that ultrasound waves emitted by the ultrasound sensor travel in the direction or opposite the direction of a fluid flowing through the flow-through element when in fluid communication with a line or when being an integral part of a line, in particular an IV line. In this example, changes in fluid velocity also influence the detected ultrasound intensity. In an alternative example, ultrasonic source and sensor are placed perpendicular to a fluid-flow direction along the flow-through element, such that ultrasound waves emitted by the ultrasound sensor travel in a plane perpendicular to the direction of a fluid flowing through the flow-through element when in fluid connection with a line. In this example, the fluid velocity does not influence the intensity detected by the ultrasound sensor.

According to one aspect, the system comprises a communication unit for establishing a communication connection between the system and a pump for use with a line of a medical fluid delivery system. In this way, information on the detected volume or volume change and inferred pressure and pressure change, respectively, can be communicated to the system and the pump. In this way, an external system in communication with a pump of the medical fluid delivery system, in particular an IV pump, is provided.

A further subject of the present invention is a line of a medical fluid delivery system, wherein the line comprises a flow-through element of the system according to one of the preceding claims 1 to 10, wherein the flow-through element is an integral part of the line of a medical fluid delivery system.

Pump for use with a line of a medical fluid delivery system, in particular a line according to claim 12, wherein the pump comprises an ultrasonic measurement unit of a system according to one of the preceding claims 1 to 10.

A further subject of the present invention is a method for monitoring pressure in a line of a medical fluid delivery system, in particular in an IV line, using a system according to claims 1 to 11 or a line according to claim 12 and a pump according to claim 13. The method comprises the steps of detecting a current probe volume and/or detecting a change in probe volume using the ultrasonic measurement unit. From the current probe volume, a current pressure inside a line of a medical fluid delivery system, in particular in an IV system, can be inferred when the flow-through element is in fluid communication with the line or an integral part of the line , in particular the IV line. Likewise, a detected change in probe volume is indicative of a change in pressure in the line, in particular the IV line.

In one aspect, the current probe volume and/or the change in probe volume are detected by the ultrasonic measurement unit through detection of a time delay in ultrasonic pulse emitted by an ultrasonic source of the ultrasonic measurement unit. The time delay is detected by an ultrasonic sensor of the ultrasonic measurement unit. Alternatively or additionally, the current probe volume and/or the change in probe volume are detected by the ultrasonic measurement unit through detection of a change in amplitude of an ultrasonic wave emitted by an ultrasonic source of the ultrasonic measurement unit. The change in amplitude is detected by an ultrasonic sensor of the ultrasonic measurement unit.

The idea underlying the invention shall subsequently be described in more detail with respect to the embodiment shown in the drawings. Herein:
- Fig. 1: illustrates a system and a method for monitoring pressure in a line of a medical fluid delivery system according to a first example;
- Fig. 2: illustrates a system and a method for monitoring pressure in a line of a medical fluid delivery system according to a second example;
- Fig. 3: illustrates a system and a method for monitoring pressure in a line of a medical fluid delivery system according to a third example; and
- Fig. 4: illustrates a system and a method for monitoring pressure in a line of a medical fluid delivery system according to a fourth example; and
- Fig. 5: illustrates a system and a method for monitoring pressure in a line of a medical fluid delivery system according to a fifth example.

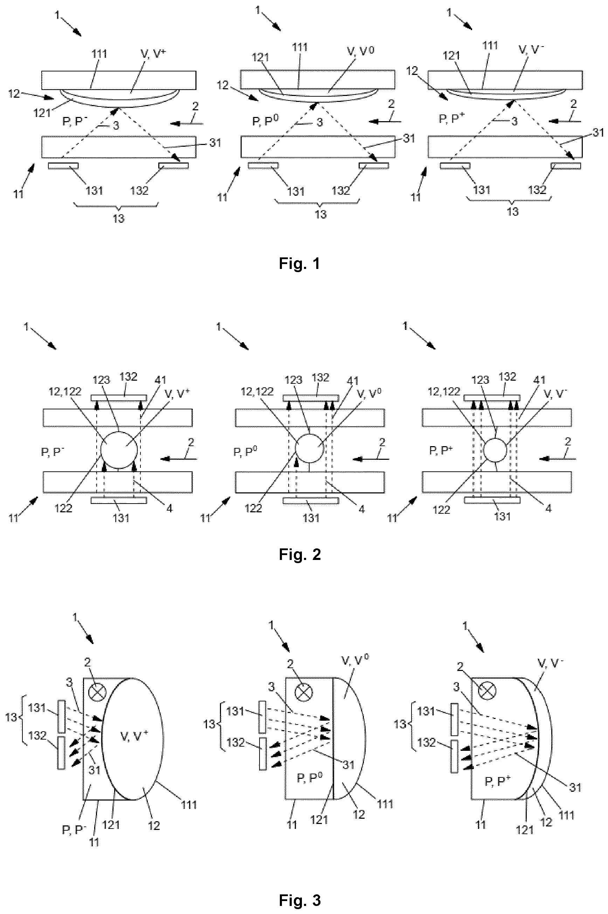

Fig. 1 illustrates a system 1 and a method for monitoring pressure in a line of a medical fluid delivery system, in particular in an IV line, according to a first example. The left, middle and right panels illustrate the system 1 and method at three different pressures P⁻, P⁰ and P⁺ in the system 1, which is indicative of a pressure in a line of a medical fluid delivery system, in particular an IV line, in fluid communication with the system 1. First, the middle panel with an intermediate pressure P° in the system 1 is being described. The system 1 comprises a flow-through element 11 which is to be brought into fluid communication with a line of a medical fluid delivery system in order to monitor the pressure in the line. When brought into fluid communication with the line, a fluid passing through the line flows through the flow-through element 11 in a fluid-flow direction 2. The flow-through element 11 has a fixed volume and is, for example, a tube or pipe or tube or pipe segment. The flow-through element 11 has, for example, a circular cross section with a diameter matching the diameter of a tubing of the line of the medical fluid delivery system. In this way, pressure artefacts due to changes in diameter along the fluid-flow direction are minimized. Inside the flow-through element 11, a probe 12 is provided. The probe 12 has a probe volume V which is responsive the pressure P in the flow-through element 11, which in turn is indicative of the pressure in the line where the system 1 is placed. The probe volume V is bounded in part by an elastic membrane 121 and in part by an inner surface 111 of the flow-through element 11. Moreover, the probe volume V is filled with a compressible medium. At pressure P⁰, the probe volume V is V⁰. Due to the elastic membrane 121, pressure P in the flow-through element 11 results in a force on the compressible medium to which the compressible medium reacts with a volume change, resulting in a change of probe volume V. Hence, the probe volume V is responsive to the pressure P in the flow-through element 11. An ultrasonic measurement unit 13 is part of the system 1. The ultrasonic measurement unit 13 detects a current probe volume. Alternatively or additionally, the ultrasonic measurement unit 13 detects a change in the probe volume, e.g. using the intermediate pressure P° as a reference value. The ultrasonic measurement unit 13 is arranged on the exterior of the flow-through element 11. In this way, fluid contact between the ultrasonic measurement unit 13 and a fluid in the flow-through element 11 is prevented. The ultrasonic measurement unit 13 comprises an ultrasonic source 131, or emitter, for emitting ultrasonic sound and an ultrasonic sensor 132, or detector, for detecting ultrasonic sound. In the depicted example, the ultrasonic source 131 is an emitter of ultrasonic pulses and the ultrasonic sensor 132 is a sensor for detecting travel times, in particular changes in travel times, of ultrasonic pulses emitted by the source 131. In particular, the ultrasonic source 131 emits ultrasonic pulses 3 towards the probe 12, in particular towards the elastic membrane 121 of the probe 12. These pulses 3 are being reflected by the probe 12, in particular its membrane 121. The reflected pulses 31 are detected by the ultrasonic sensor 132. In particular, the ultrasonic measurement unit 13 detects the travel time of the detected pulse 31 from emitter 131 to sensor 132.

For example, the ultrasonic source 131 and the sensor 132 are arranged on the same side of the flow-through element 11, for example along a direction 2 of fluid flow. In this case, fluid flow influences the ultrasonic travel times. In particular, the source 131 and sensor 132 are arranged opposite to the probe 12 on an exterior of the flow-through element 11.

If the pressure in the line decreases, the pressure P in the flow-through element 11 decreases, resulting e.g. in a pressure P⁻ lower than the intermediate pressure P°. This is depicted in the left panel. As result, the force exerted by the fluid pressure on the probe 12 is reduced. The compressible medium inside the probe 12 extends. The probe volume V increases to a larger volume V⁺ than the intermediate volume V⁰. The distance travelled by an ultrasonic pulse 31 from source 131 to sensor 132 is reduced. As a result, the travel time is shorter. Hence, from travel-time measurements or time-delay measurements of the ultrasonic measurement unit 13, the probe volume V and hence the pressure in the line can be inferred.

Similarly, when the pressure in the line increases, the pressure P in the flow-through element 11 increases, resulting e.g. in a pressure P⁺ higher than the intermediate pressure P°. This is depicted in the right panel. As a result, the force on the elastic membrane 121 increases, the probe volume V is reduced to a lower value V⁻. The distance travelled by an ultrasonic pulse 31 from source 131 to sensor 132 increases and so does the travel time.

In this way, a system 1 is provided that allows pressure monitoring along a line of a medical fluid delivery system. In particular, the flow-through element 11 is free of any electronic parts, and hence, does not require a power source. It is able to withstand standard internal pressures of medical fluid delivery systems and can be flexibly inserted at multiple locations along the line. Moreover, it does not have to be disposed together with the line, saving costs and waste.

Fig. 2 illustrates a system 1 and method for monitoring pressure in a line of a medical fluid delivery system, in particular an IV line, according to an alternative example. In the following, only the differences to the system 1 and method described with regard to the example of Fig. 1 are being described. Again, in the left, middle and right panels, the system 1 is depicted at different pressure values P⁻, P° and P⁺. In the depicted example, the entire probe volume V is bounded by an elastic membrane 121. The elastic membrane 121 forms a balloon 122. The probe 12 surface which is responsive to the pressure P in the flow-through element 11 is hence larger than in the example of Fig. 1. The probe 12 is fixed to the flow-through element 11 through fixation elements 123, for example straps connecting the balloon 122 to the flow-through element 11. This restricts the movement of the probe 12 relative to the ultrasonic measurement unit 13 when a fluid flow is present. In the present example, the ultrasonic measurement unit 13 also comprises a source 131 and a sensor 132. In this example, however, the measurement unit 13 is configured to measure the amplitude and hence the intensity of ultrasonic waves emitted by the source 131 and reaching the sensor 132. In particular, the source 131 and sensor 132 are placed on opposite sides of the probe 12. Hence, ultrasonic waves 4 emitted by the source 131 either transmit the flow-through element 11 (including fluid contained in it) or are being reflected or absorbed by the probe 12. Hence, the amplitude and hence the intensity detected on the sense 132 is indicative of the probe volume V and hence of the pressure P in the flow-through element 11 and hence in the line. As shown in the middle panel, at an intermediate pressure P⁰, the probe occupies a volume V⁰. Hence, a certain part of the ultrasonic waves 4 emitted by the source 131 is absorbed or reflected by the probe 12 and, hence, does not reach the sensor 132.

If the in-line pressure decreases, as shown in the left panel, the pressure P in the flow-through element 11 decreases to a value P⁻ lower than the intermediate pressure P°. As a consequence, the compressible medium inside the probe 12, in particular the balloon 122, extends. As the probe 12 now occupies a larger volume V⁺ in the travel path of the ultrasonic waves 4, more waves are prevented from reaching the sensor 132. Hence, the intensity detected at the sensor 132 decreases. Similarly, if the in-line pressure increases, the pressure P in the flow-through element 11 increases to a value P⁺ larger than the intermediate value P°. Consequently, the probe volume V decreases to a value V⁻. Hence, the amplitude and hence the intensity detected at the sensor 132 increases.

Fig. 3 illustrates a system 1 and method for monitoring pressure in a line of a medical fluid delivery system, in particular an IV line, according to an alternative example. In the following, only the differences to the system 1 and method described with regard to the example of Fig. 1 are being described. The present example differs in the arrangement of the ultrasonic measurement unit 13 relative to the fluid-flow direction 2. Here, the ultrasonic measurement unit 13 is placed such that ultrasonic pulses 3 emitted by the source 131 and detected by the sensor 132 travel in a plane perpendicular to the fluid-flow direction 2. In this way, the influence of fluid velocity and fluid-flow direction on the travel time of the ultrasonic pulses is reduced. Moreover, in the present example, the flow-through element 11 has a D-shaped cross section. The ultrasonic measurement unit 13 is arranged on the exterior of the flow-through element 11 on the flat side of the flow-through element 11. In this way, unwanted reflection and refraction effects of the ultrasound on the outer surface of the flow-through element 11 are reduced. Ultrasonic waves can easily be coupled into the flow-through element 11. Moreover, the curved section of the flow-through element 11 increases the probe volume V. Again, the left, middle and right panel depict the system 1 at three different pressure values P⁻, P⁰ and P⁺ in the flow-through element 11 and with the corresponding three different probe volumes V⁺, V⁰ and V⁻.

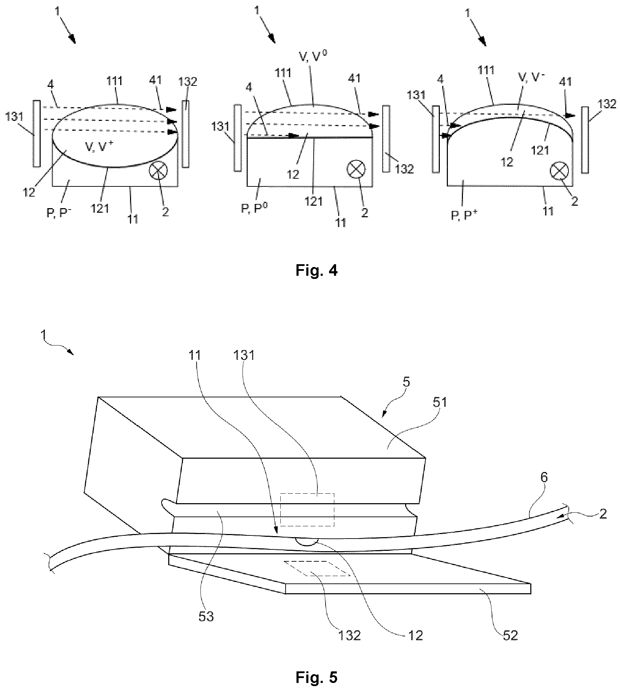

Fig. 4 shows a further example of a system 1 and method for monitoring pressure in a line of a medical fluid delivery system, in particular an IV line, according to an alternative example. In the following, only the differences to the system 1 and method described with regard to the example of Fig. 3 are being described. The present example differs from the example of Fig. 3 in the ultrasonic measurement unit 13. As in the example of Fig. 2, here, source 131 and sensor 132 are placed on opposite sides of the flow-through element 11. The ultrasonic measurement unit 13 is configured to measure the intensity of ultrasonic waves emitted by the source 131 and detected by the sensor 132. As the probe 12 is arranged between source 131 and sensor 132, the intensity is indicative of the probe volume V and hence, of the pressure P in the flow-through element 11 and hence, of the in-line pressure. Again, the left, middle and right panel depict the system 1 at three different pressure values P⁻, P⁰ and P⁺ in the flow-through element 11 and with the corresponding three different probe volumes V⁺, V⁰ and V⁻.

Fig. 5 shows a further example of a system 1 and method for monitoring pressure in a line 6 of a medical fluid delivery system, in particular an IV line, according to an alternative example. In the following, only the differences to the systems of the examples described in Figures 1 to 4 are described. In the depicted example, the system 1 comprises a flow-through element 11 being an integral part of the line 6. The flow-through element 11 comprises a probe 12. For example, an elastic membrane 121 bounds the probe volume V at least partly, wherein the remaining part of the probe volume V is bounded by an inner surface 111 of the flow-through element 11, here also an inner surface 111 of the line 6. However, all other probes 12 described with regard to the previous examples can be arranged inside the flow-through element 11. The ultrasonic measurement unit 13 is part of a pump 5 for use with the line 6 of a medical fluid delivery system of which the flow-through element 11 is an integral part. Both examples, an ultrasound measurement unit 13 for measuring delay times as well as an ultrasound measurement unit 13 for measuring ultrasound intensities, i.e. amplitudes, can be provided inside the pump 5. The pump 5 comprises a housing 51. The housing 51 comprises a receptacle 53, e.g. a recess formed in the housing, for the line 6. The receptacle 53 can be opened and closed with a closing element 52, e.g. a door, of the pump 5. The ultrasound source 131 is, for example, arranged in in the pump housing 51, in particular along the receptacle 53 in the pump housing 51. The ultrasound source 131 is arranged inside the housing 51, in particular along the receptacle 53, such that ultrasound waves and/or ultrasound pulses can be emitted from the ultrasound source 131 towards the flow-through element 11, in particular towards the probe 12. An ultrasound sensor 132 is arranged in the door 52 such that it rests opposite the ultrasound source 131 when the door 52 is closed. Ultrasound waves and/or ultrasound pulses emitted by the source 131 towards the flow-through element 11, in particular towards the probe 12 inside the flow-through element 11, can be detected by the ultrasound sensor 132. Alternatively, the position of ultrasound source 131 and ultrasound sensor 132 are reversed. In this alternative, the ultrasound sensor 132 is arranged in the pump housing 51, in particular along the receptacle 53 for the line 6 and the ultrasound source 131 is arranged in the closing element 52, in particular, inside the door, such that it rests opposite to the ultrasound sensor 132, when the closing element 52 is in its closed position. In this way, expensive and electronic components of the system 1 are arranged inside the pump 5. They are protected and can be reduced, as they are not in contact with medical fluid inside the line 6 or with a patient. The flow-through element 11 as well as the probe 12 do not contain any expensive and electronic parts. They are part of the line 6, which is, for example, a disposable. In this way, an hygienic and cheap system and method for monitoring pressure in a line 6 is provided.

### List of Reference Numerals

- 1: system
- 11: flow-through element
- 111: inner surface
- 12: probe
- 121: elastic membrane
- 122: balloon
- 123: fixation element
- 13: ultrasound measurement unit
- 131: ultrasound source
- 132: ultrasound sensor
- 2: fluid-flow direction
- 3: emitted ultrasound pulse
- 31: reflected ultrasound pulse
- 4: emitted ultrasound wave
- 41: transmitted ultrasound wave
- 5: pump
- 51: pump housing
- 52: closing element
- 53: recess
- 6: line
- P⁰, P⁺, P⁻: pressure
- V⁰, V⁺, V⁻: probe volume

## Claims

1. A system (1) for monitoring pressure in a line of a medical fluid delivery system, **characterized in that** the system (1) comprises:
- a flow-through element (11) to be brought into fluid communication with a line of a medical fluid delivery system or a flow-through element (11) being an integral part of a line (6) of a medical fluid delivery system, wherein the volume of the flow-through element (11) is fixed;
- a probe (12) provided inside the flow-through element (11), wherein the probe volume (V) is responsive to a pressure (P) in the flow-through element (11); and
- an ultrasonic measurement unit (13) for detecting a current probe volume (V) and/or detecting a change in probe volume (V).

2. The system (1) according to claim 1, **characterized in that** at least a part of the probe volume (V) is bounded by an elastic membrane (121).

3. The system (1) according to claim 2, **characterized in that** the entire probe volume (V) is bounded by an elastic membrane (121) forming a balloon (122).

4. The system (1) according to claim 3, **characterized in that** the probe (12) comprises fixation elements (123) for fixing the probe (12) to the flow-through element (11).

5. The system (1) according to claim 2, **characterized in that** at least part of the probe volume (V) is bounded by an inner surface (111) of the flow-through element (11).

6. The system (1) according to any of the preceding claims, **characterized in that** the probe volume (V) is filled with a compressible medium.

7. The system (1) according to any of the preceding claims, **characterized in that** the flow-through element (11) is a tube.

8. The system (1) according to any of the preceding claims, **characterized in that** the ultrasonic measurement unit (13) comprises an ultrasonic source (131) and an ultrasonic sensor (132).

9. The system (1) according to claim 8, **characterized in that** the ultrasonic measurement unit (13) is configured to measure the travel time of ultrasonic pulses emitted by the ultrasonic source (131) towards the flow-through element (11) and detected by the ultrasonic sensor (132) or that the ultrasonic measurement unit (13) is configured to measure the amplitude of ultrasonic waves emitted by the ultrasonic source (131) towards the flow-through element (11) and detected by the ultrasonic sensor (132).

10. The system (1) according to any of the preceding claims, **characterized in that** the ultrasonic measurement unit (13) is part of a pump (5) for use with a line (6) of a medical fluid delivery system.

11. The system (1) according to one of the preceding claims 1 to 9, **characterized in that** the ultrasonic measurement unit (13) is arranged on an exterior of the flow-through element (11).

12. Line (6) of a medical fluid delivery system, **characterized in that** the line (6) comprises a flow-through element (11) of the system (1) according to one of the preceding claims 1 to 10, wherein the flow-through element (11) is an integral part of the line (6) of a medical fluid delivery system.

13. Pump (5) for use with a line (6) of a medical fluid delivery system, **characterized in that** the pump (5) comprises an ultrasonic measurement unit (13) of a system (1) according to one of the preceding claims 1 to 10.

14. Method for monitoring pressure in a line of a medical fluid delivery system using a system (1) according to claims 1 to 11, **characterized in that** the method comprises the steps of:
- detecting a current probe volume (V) and/or detecting a change in probe volume (V) using the ultrasonic measurement unit (13).

15. Method according to claim 14, **characterized in that** the current probe volume (V) and/or the change in probe volume (V) are detected by the ultrasonic measurement unit (13) through detection, by an ultrasonic sensor (132) of the ultrasonic measurement unit (13), of a delay in a signal emitted by an ultrasonic source (131) of the ultrasonic measurement unit (13) and/or through detection, by an ultrasonic sensor (132) of the ultrasonic measurement unit (13), of a change in amplitude of a signal emitted by an ultrasonic source (131) of the ultrasonic measurement unit (13).
